(19) Europäisches Patentamt
European Patent Office
Office européen des brevets

(11) Publication number: 0 439 174 A2

(12) **EUROPEAN PATENT APPLICATION**

(21) Application number: 91100912.4

(22) Date of filing: 24.01.91

(51) Int. Cl.5: C07K 7/02, A61K 37/43

The applicant has subsequently filed a sequence listing and declared, that it includes no new matter.

(30) Priority: 25.01.90 US 470492

(43) Date of publication of application: 31.07.91 Bulletin 91/31

(84) Designated Contracting States: AT BE CH DE DK ES FR GB GR IT LI LU NL SE

(71) Applicant: MERRELL DOW PHARMACEUTICALS INC.
2110 East Galbraith Road
Cincinnati Ohio 45215(US)

(72) Inventor: Krstenansky, John L.
3749 Ault Park
Cincinnati, Ohio 45208(US)

(74) Representative: Vossius & Partner
Siebertstrasse 4 P.O. Box 86 07 67
W-8000 München 86(DE)

(54) **Inhibition of gut endothelium ion-secretion by peptide derivatives.**

(57) Certain peptide derivatives inhibit ion secretion from gut endothelium and are thus useful in treating diarrhea especially the secretory diarrhea and the diarrheas associated with carcinoid syndrome and VIPomas.

EP 0 439 174 A2

# INHIBITION OF GUT ENDOTHELIUM ION-SECRETION BY PEPTIDE DERIVATIVES

This invention relates to peptide derivatives which inhibit ion-secretion from gut endothelium. These peptide derivatives are useful in the treatment of diarrhea, particularly diarrhea associated with carcinoid syndrome, inflammatory bowel disease, and VIPomas.

Somatostatin has been reported to be useful in the treatment of patients with certain pituitary adenomas, insulinomas, glucagonomas, carcinoid syndrome, and vasoactive intestinal peptide-secreting tumors (VIPomas) as well as a variety of other conditions. However, somatostatin therapy has been limited, primarily because of very short serum half-life of this naturally occurring peptide. Recently the synthetic peptide octreolide acetate was developed. This compound possesses many of the biological activities of natural somatostatin but overcomes many of its limitations. Octreotide has been approved by the USFDA for use in the treatment of diarrhea associated carcinoid syndrome and VIPomas. The use of somatostatin and its analogs suffers from the fact that somatostatin-like agents possess a large array of pharmacologic activities which include inhibition of the secretion of insulin, glucagon, pancreatic polypeptide, gastrin, gastric inhibitory peptide, motilin, neurotensin, secretin, cholecystokinin, pancreatic trypsin and pancreatic amylase, a decrease in fat absorption and a delay in carbohydrate absorption (M.D. Katz and B.L. Erstad, Clinical Pharmacy 8: 255-273 (1989)). A material more selective in its actions would be desirable.

Neuropeptide Y is known to produce a net uptake of chloride ion in intestinal epithelial cells (K.A. Hubel and K.S. Renquist, J. Pharmacol. & Exp. Ther. 238: 167-169 (1986) and H.M. Cox et al., J. Physiology 398: 65-80 (1988)) and inhibit secretagogue evoked chloride transport (H.M. Cox and A.W. Cuthbert, Pflugers Arch. 413: 38-42 (1988)). In the case of IP, NPY is thought to inhibit VIP's actions by inhibiting cAMP production that would be induced by VIP (A.L. Servin et al., Endocrinology 124: 692-700 (1989)). The use of Neuropeptide Y itself to achieve this effect *in vivo* suffers from the vascular effects of Neuropeptide Y. The analogs described in this patent have selective action on the gut with little to none of the detrimental vascular effects of the native hormone. Applicant has discovered that certain peptide derivatives which were previously reported to posses agonist activity against the neuropeptide Y receptor also inhibit ion secretion from gut endothelium. Such compounds would be beneficial in the treatment of diarrhea particularly the diarrheas associated with carcinoid syndrome and VIPomas.

Peptide derivatives of formula 1

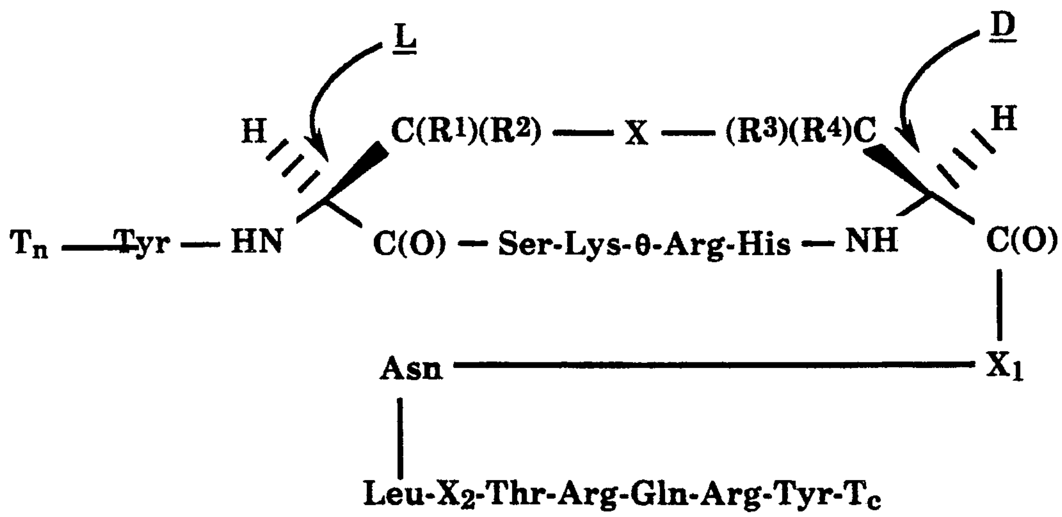

1

wherein $X_1$ is Leu, Ile, Met, Nle, or Val;
$X_2$ is Leu, Ile, Met, Nle, or Val;
$R^1$, $R^2$, $R^3$, and $R^4$ are each independently selected from a hydrogen and $(C_1-C_4)$alkyl;
$T_n$ is $(C_2-C_{10})$acyl, $(C_1-C_4)$alkyl;
$T_c$ is OR' or NHR';
wherein R' is a hydrogen or a $(C_1-C_4)$alkyl group;
$\theta$ is a group of the structural formula

$-NH-(CH_2)_n-CO_2-$;

wherein n is an integer of from 1-11; and
X is a bond or is a group of the formulae

$$-S-S-,$$

$$-S-,$$

$$-O-,$$

$$-S-(C(R^5)(R^6))_m-S-,$$

$$-O-(C(R^5)(R^6))_m-O-,$$

$$-S-(C(R^5)(R^6))_m-O-,$$

$$-O-(C(R^5)(R^6))_m-S-,$$

$$-C(=O)-N(R')-,$$

$$-N(R')-C(=O)-,$$

$$-C(=O)-O-,$$

$$-O-C(=O)-,$$

$$C(=O)-S-,$$

$$-S-C(=O)-, \text{ and}$$

$$-N(H)-C(=O)-N(H)-$$

wherein $R^5$ and $R^6$ are each independently selected from the group consisting of hydrogen and $(C_1-C_4)$alkyl and m is an integer of a value from 1 to 3
and the pharmaceutically acceptable salts thereof inhibit ion-secretion from gut endothelium and are useful in the treatment of diarrhea particularly diarrhea associated with carcinoid syndrome and VIPomas.

The following common abbreviations of the amino acids and amino and carboxy terminal groups are used throughout this specification:

| | |
|---|---|
| Ala | - alanine |
| Val | - valine |
| Leu | - leucine |
| Ile | - isoleucine |
| Pro | - proline |
| Met | - methionine |
| Ser | - serine |
| Thr | - threonine |
| Cys | - cysteine |
| cys | - D-cysteine |
| Tyr | - tyrosine |
| Asn | - asparagine |
| Asp | - aspartic acid |
| Lys | - lysine |
| Arg | - arginine |
| His | - histidine |

| | |
|---|---|
| Glu | - glutamate |
| Nle | - norleucine |
| Aoc | - 8-aminooctanoic acid |
| # - | $-NH_2$ |

An alkyl group is taken to include straight, branched, or cyclic alkyl groups, for example, methyl, ethyl, propyl, isopropyl, butyl, isobutyl, tert-butyl, pentyl, isopentyl, sec-pentyl, cyclopentyl, hexyl, isohexyl, cyclohexyl and cyclopentylmethyl. An acyl group of from 2 to 10 carbon atoms is taken to include straight, branched, cyclic, saturated and unsaturated acyl groups having 1 or 2 carbonyl moieties per group, for example, acetyl, benzoyl succinyl, maleyl, and glutaryl. Those peptides wherein the amino group of the amino terminal amino acid is substituted with two alkyl or acyl groups are also considered to be within the scope of the peptides of this invention.

The natural amino acids, with the exception of glycine, contain a chiral carbon atom. Unless otherwise specifically indicated, the optically active amino acids, referred to herein, are of the L-configuration. Notably, the carbon atom which corresponds to the α-carbon atom of the eighth α-amino acid from the amino terminal end of the formula 1 peptides must be of the D-configuration to permit cyclyzation. This carbon atom is indicated by a "D".

The polypeptides of formula 1 can form pharmaceutically acceptable salts with any non-toxic, organic or inorganic acid. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic and sulfonic acids such as methane sulfonic acid and 2-hydroxyethane sulfonic acid. Salts of the carboxy terminal amino acid moiety include the non-toxic carboxylic acid salts formed with any suitable inorganic or organic bases. Illustratively, these salts include those of alkali metals, as for example, sodium and potassium; alkaline earth metals, such as calcium and magnesium; light metals of Group IIIA including aluminum; and organic primary, secondary and tertiary amines, as for example, trialkylamines, including triethylamine, procaine, dibenzylamine, l-ethenamine, N,N'-dibenzylethylenediamine, dihydroabietylamine, N-(lower)alkylpiperidine, and any other suitable amine.

As with any generic group of chemical compounds, certain groups are preferred. Applicants prefer those peptide derivatives of formula 1 wherein $X_1$ is isoleucine. Applicants also prefer those peptide derivatives of formula 1 wherein $X_2$ is isoleucine. Especieally preferred are those peptide derivatives of formula 1 wherein $x_1$ and $X_2$ are each an isoleucine. Also preferred are those peptide derivatives wherein Tc is $NH_2$ and wherein $\theta$ is Aoc. Also preferred are those compounds wherein $R^1$, $R^2$, $R^3$, and $R^4$ are each hydrogen and X is a -S-S- group. The most preferred peptide derivative of formula 1 is the peptide derivative of formula 2.

**Tyr-Cys-Ser-Lys-Aoc-Arg-His-Cys-Ile-Asn-Leu-Ile-Thr-Arg-Gln-Arg-Tyr-$NH_2$ 2**

The proteins of this invention can be prepared by a variety of procedures readily known to those skilled in the art. Such procedures include the solid phase sequential procedure which can be performed using established automated methods such as by use of an automated peptide sythesizer.

The resin support employed can be any suitable resin conventionally employed in the art for the solid phase preparation of polypeptides, preferably polystyrene which has been cross-linked with from 0.5 to about 3 percent divinyl benzene, which has been either converted to the p-methylbenzhydrylamine or benzhydrylamine derivative (for C-terminal amides) or chloromethylated or hydroxymethylated to provide sites for ester formation with the initially introduced α-amino protected amino acid (for producing C-terminal alkylamides and esters).

An example of a hydroxymethyl resin is described by Bodanszky, et al., Chem. Ind. (London) 38, 1597-98 (1966). A chloromethylated resin is commercially available from Bio Rad Laboratories, Richmond, California, and the preparation of such a resin is described by Stewart et al., "Solid Phase Peptide Synthesis" (Freeman & Co., San Francisco 1969), Chapter 1, pp. 1-6. The protected amino acid can be bound to the resin by the procedure of Gisin, Helv. Chem Acta, 56, 1476 (1973). Many resin bound, protected amino acids are commercially available. As an example, to prepare a polypeptide of this invention

wherein the carboxy terminal end is a Thr residue, a tert-butyloxycarbonyl (Boc) protected Thr bound to a benzylated, hydroxymethylated phenylacetamidomethyl (PAM) resin can be used and is commercially available.

Following the coupling of the α-amino protected amino acid to the resin support, the protecting group is removed using any suitable procedure such as by using trifluoroacetic acid in methylene chloride, trifluoroacetic acid alone, or HCl in dioxane. The deprotection is carried out at a temperature of between 0 °C and room temperature. Other standard cleaving reagents and conditions for removal of specific α-amino protecting groups may be used. After removal of the α-amino protecting group the other amino protected amino acids are coupled step-wise in the desired order. Alternatively, multiple amino acid groups may be coupled by the solution method prior to coupling with the resin supported amino acid sequence.

The α-amino protecting group employed with each amino acid introduced into the polypeptide sequence may be any such protecting group known to the art. Among the classes of α-amino protecting groups contemplated are (1) acyl type protecting groups such as: formyl, trifluoroacetyl, phthalyl, toluenesulfonyl (tosyl), benzenesulfonyl, nitro-phenylsulfenyl, tritylsulfenyl, o-nitrophenoxyacetyl and α-chlorobutyryl; (2) aromatic urethan type protecting groups such as benzyloxycarbonyl and substituted benzyloxycarbonyl, such as p-chlorobenzyloxycarbonyl, p-nitrobenzyl- carbonyl, p-bromobenzyl-oxycarbonyl, p-methoxybenzyloxycarbonyl, 1-(p-biphenylyl)-1-methylethoxycarbonyl, α, α-dimethyl-3,5-dimethoxybenzyloxycarbonyl, benzhydryloxycarbonyl and 9-fluorenylmethoxycarbonyl (Fmoc); (3) aliphatic urethan protecting groups such as tert-butyloxycarbonyl (Boc), diisopropylmethoxycarbonyl, isopropyloxycarbonyl, ethoxycarbonyl and allyloxycarbonyl; (4) cycloalkyl urethan type protecting groups such as cyclopentyloxycarbonyl, adamantyloxycarbonyl and cyclohexyloxycarbonyl; (5) thio urethan type protecting groups such as phenylthiocarbonyl; (6) alkyl type protecting groups such as triphenylmethyl (trityl) and benzyl; and (7) trialkylsilane groups such as trimethylsilane. The preferred α-amino protecting group is tert-butyloxycarbonyl or Fmoc.

The selection of an appropriate coupling reagent is within the skill of the art. A particularly suitable coupling reagent where the amino acid to be added is Gln, Asn or Arg is N,N'-diisopropylcarbodiimide and 1-hydroxy-benzotriazole. The use of these reagents prevents nitrile and lactam formation. Other coupling agents are (1) carbodiimides (e.g., N,N'-dicyclohexylcarbodiimide and N-ethyl-N'-(γ-dimethylaminopropylcarbodiimide); (2) cyanamides (e.g., N,N-dibenzylcyanamide); (3) ketenimines; (4) isoxazolium salts (e.g., N-ethyl-5-phenylisoxazolium-3'-sulfonate; (5) monocyclic nitrogen containing heterocyclic amides of aromatic character containing one through four nitrogens in the ring such as imidazolides, pyrazolides, and 1,2,4-triazolides. Specific heterocyclic amides that are useful include N,N'-carbonyldiimidazole and N,N-carbonyl-di-1,2,4-triazole; (6) alkoxylated acetylene (e.g., ethoxyacetylene); (7) reagents which form a mixed anhydride with the carboxyl moiety of the amino acid (e.g., ethylchloroformate and isobutylchloroformate) or the symmetrical anhydride of the amino acid to be coupled (e.g., $(Boc-Ala)_2-O$) and (8) nitrogen containing heterocyclic compounds having a hydroxy group on one ring nitrogen (e.g., N-hydroxyphthalimide, N-hydroxysuccinimide and 1-hydroxybenzotriazole). Other activating reagents and their use in peptide coupling are described by Kapoor, J. Pharm. Sci., 59, pp. 1-27 (1970). Applicants prefer the use of the symmetrical anhydride as a coupling reagent for all amino acids except Arg, Asn and Gln.

Each protected amino acid or amino acid sequence is introduced into the solid phase reactor in about a four-fold excess and the coupling is carried out in a medium of dimethylformamide: methylene chloride (1:1) or in dimethylformamide alone or preferably methylene chloride alone. In cases where incomplete coupling occurs, the coupling procedure is repeated before removal of the α-amino protecting group, prior to the coupling of the next amino acid in the solid phase reactor. The success of the coupling reaction at each stage of the synthesis is monitored by the ninhydrin reaction as described by E. Kaiser et al, Analyt. Biochem. 34, 595 (1970).

After the desired amino acid sequence has been obtained, the peptide is removed from the resin. This can be done by hydrolysis such as by treatment of the resin bound polypeptide with a solution of dimethyl sulfide, p-cresol and thiocresol in liquid hydrofluoric acid.

As is known in the art of solid phase peptide synthesis many of the amino acids bear functionalities requiring protection during the chain preparation. The use and selection of the appropriate protecting group is within the ability of those skilled in the art and will depend upon the amino acid to be protected and the presence of other protected amino acid residues on the peptide. The selection of such a side chain protecting group is critical in that it must be one which is not removed during cleavage of the protecting group of the α-amino moiety. For example, suitable side chain protecting groups for lysine are benzyloxycarbonyl and substituted benzyloxycarbonyl, said substituent being selected from halo (e.g., chloro, bromo, fluoro) and nitro (e.g., 2-chlorobenzyloxycarbonyl, p-nitrobenzyloxy-carbonyl, 3,4-dichlorobenzyloxycarbonyl), tosyl, t-amyloxycarbonyl, t-butyloxycarbonyl and diisopropylmethoxycarbonyl. The alcoholic

hydroxyl group of threonine and serine can be protected with an acetyl, benzoyl, tert-butyl, trityl, benzyl, 2,6-dichlorobenzyl or benzyloxycarbonyl group. The carboxylic group of Aspartic acid and Glutamic acid can be protected with a benzyl or cyclohexyl group. The preferred protecting group is benzyl.

These groups can be removed by procedures well known in the art. Typically protecting group removal is done after the peptide chain synthesis is complete but the protecting groups can be removed at any other appropriate time.

In general, the cyclized peptides are prepared from an appropriate precursor linear peptide derivative either prior to or after removal of the peptide from the support. The compounds of formula 1 wherein X is a -S-S- group are prepared from the corresponding free sulfhydryl-containing, linear peptides by well known oxidative coupling technics such as by oxidizing the linear peptide with potassium ferricyanide described in, for example, Stewart et al., "Solid Phase Peptide Synthesis" (Freeman & Co., San Francisco 1969), Chapter 1, p. 95. The compounds of formula 1 wherein X is a -S-Alk-S-, -O-Alk-O-, -O-Alk-S-, or -S-Alk-O- group wherein Alk is an ethylene can be prepared from the free sulfhydryl-containing and/or alcohol-containing linear peptides by reaction with a 1,2-dibromo derivative of an appropriate acyclic or cyclic, saturated or insaturated alkyl in a manner analogous to that described in H.I. Mosberg and J.R. Omnaas, J. Amer. Chem. Soc. 107, 2986-2987 (1985). The compounds of formula 1 wherein X is a -S-Alk-S- group wherein Alk is a methylene group are prepared by reaction of the free sulfhydryl-containing linear peptide with an appropriate acyclic or cyclic, saturated or unsaturated alkyl ketone or aldehyde in a manner analogous to that described in J. Amer. Chem. Soc. 76, 1945 (1954). The preparation of those compounds of formula 1 wherein X is an -O-or -S- group can be accomplished in the manner set forth in K. Jost, Collect. Czech. Chem. Commun. 36, 218 (1971) and in the United States Patent Number 4,161,521.

The compounds of formula 1 wherein X is -C(=O)N(R) or - N(R)C(=O)- are cyclic lactams and are obtained by side-chain coupling as described by Schiller et al., J. Med. Chem. 28:1766-1771 (1985) or by Al-Oblidi et al., J. Med. Chem. 32:2555-2561 (1989). The compounds of formula 1 wherein X is a -C(=O)O-, -OC(=O)-, -C(=O)S-, or -SC(=O)- group are depsipepties and thiodepsipeptides. These compounds can be prepared by esterification of the linear peptide analog with a coupling reagent (e.g., 1-ethyl-3-(3-dimethylaminopropyl) carbodiimide). See R.K. Olsen and M.K. Dhaon, Peptides:Synthesis Structure-Function, D.H. Rich & E. Gross (eds.) 1981, Pierce Chemical Co., Rockford, pp. 41-44. The compounds of formula 1 wherein X is -NHC(=O)-NH- are prepared by an intramolecular cyclyzation of two free amino groups in an otherwise protected peptide fragment. The free amino precursor is very slowly titrated with 1.1 equivalents of phosgene to yield the desired intramolecularly cyclized urea entity.

The ability of the peptide derivatives of formula 1 to inhibit endothelial ion secretion demonstrated by a reduction of short circuit current as described in H. Cox et al., J. Physiol 398:65-80 (1988), 2 was found to be nearly as effective as NPY itself. This is in contrast to other analogs such as C7-NPY and C5-NPY (previously described in P.N.A.S. USA 86:4377-4381 (1989) which were markedly less potent than either 2 or NPY.

By virtue of the ability of the peptide derivatives of this invention to inhibit endothelial ion secretion, the compounds would be useful in preventing diarrheas associated with disturbed ion secretion, particularly those diarrheas associated with carcinoid syndrome and with VIPomas and the secretory diarrheas commonly caused by, for example, bacterial toxins, bile acids, unabsorbed dietary fat, and some hormones such as calcitonin.

The dose of a peptide derivative of this invention required to inhibit endothelial ion secretion and therefore act as an antidiarreal agent is from 1.0 mcg/kg to 100 mcg/kg of patient body weight per day depending on the patient, the severity of the condition to be treated and the peptide derivative selected. The suitable dose for a particular patient can be readily determined. Preferably from 1 to 4 daily doses would be administered typically with from 100 mcg to 500 mcg of active compound per dose.

The term "patient" used herein is taken to mean mammals such as primates, including humans, sheep, horses, cattle, pigs, dogs, cats, rats and mice.

Although some of the peptide derivatives may survive passage through the gut following oral administration, applicants prefer non-oral administration, for example, subcutaneous, intravenous, intramuscular or intraperitoneal; administration by depot injection; by implant preparation; or by application to the mucous membranes, such as, that of the nose, throat and bronchial tubes, for example, in an aerosol can containing a peptide derivative of this invention in a spray or dry powder form.

For parenteral administration the compounds may be administered as injectable dosages of a solution or suspension of the compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable, or synthetic origin, for example, peanut oil, soybean oil, and mineral

oil. In general, water, saline, aqueous dextrose and related sugar solutions, ethanol and glycols such as propylene glycol cr polyethylene glycol are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, silicone rubber manufactured by the Dow-Corning Corporation.

## EXAMPLES

This invention is illustrated by the following, nonlimiting examples.

### EXAMPLE 1

Preparation of

Tyr-Cys-Ser-Lys-Aoc-Arg-His-cys-Ile-Asn-Leu-Ile-Thr-Arg-Gln-Arg-Tyr-$NH_2$

The peptide 2 was synthesized ona 0.5-mmol scale by solid-phase methods on p-methylbenzhydrylamine resin (0.40 mmol/g; Peptides International, Louisville, KY) using an Applied Biosystems model 430-A peptide synthesizer. All residues were double coupled using protocols supplied by the manufacturer as symmetrical anhydrides of the $N^{\alpha}$-t-butoxycarbonyl (Boc)-protected amino acids with the exception of arginine, asparagine, and glutamine that were double coupled using N,N'-dicyclohexylcarbodiimide and 1-hydroxybenzotriazole. The side-chain protection was as follows: Arg(Tos), Cys(pMeBzl), Glu(Bzl), His(Tos), Ser(Bzl), and Tyr(2-BrZ), where Tos is tosyl, Chx is cyclohexyl, pMeBzl is p-methylbenzyl, Bzl is benzyl, and 2-BrZ is 2-bromobenzyloxycarbonyl. The peptide was cleaved from the resin support and deprotected in liquid HF containing 5% (vol/vol) anisole at -5° C for 40 min. After removal of the HF *in vacuo*, the peptide was extracted from the resin with 30% (vol/vol) acetic acid and water. The extract was diluted to 1 liter; the pH was adjusted to between 8 and 9 with ammonium hydroxide and 0.01 M potassium ferricyanide was added until a yellow color persisted (≈25 ml). After stirring for 30 min., the pH was lowered to <5 with glacial acetic acid and the solution was stirred with 25 ml of settled AG 3-X4A resin (Rio-Rad) for 2 hr. The solution was filtered from the resin and lyophilized. The peptidic material that remained was purified by preparative HPLC on a Dynamax $C_{18}$ column (41.4 x 250 mm; Rainin Instruments) using acetonitrile in 0.1% trifluoroacetic acid as an eluant. The purity and identity of the peptides were assessed by analytical HPLC [Vydac 218TP54 $C_{18}$ column, 4.6 x 250 mm, 2.0 ml./min, $t_0$ = 1.9 min, linear gradient of 15-40% or 25-50% (vol/vol) acetonitrile in 0.1% trifluoroacetic acid over 25 min], by amino acid analysis [6 M HCl containing 8% (vol/vol) phenol; 106° C; 20 and 40 hr], and by fast atom bombardment-mass spectrometry (M-Scan). In addition, the cyclic analogs were shown to be negative when tested with Ellman reagent confirming the presence of the disulfide.

AAA: Asx 1.00 (1); Thr 1.01 (1); Ser 1.00 (1); Glx 1.03 (1); Ile 1.86 (2); Leu 0.99 (1); Tyr 2.02 (2); His 0.97 (1); Lys 1.10 (1); Arg 2.97 (3).

FAB-MS: $(M+H)^+$ = 2194.2 (reduced) (calcd: 2193.2) $\epsilon_{280}$ = 2821

## SEQUENCE LISTING

1. SEQ ID NO: 1:

| | |
|---|---|
| SEQUENCE TYPE: | amino acid |
| SEQUENCE LENGTH: | 17 |
| TOPOLOGY: | circular |
| MOLECULE TYPE: | peptide or protein |
| FEATURES: | Xaa at position 5 is 8-aminooctanoic acid; Cys at position 8 is in the D-configuration; Cys at position 2 and at position 8 are linked via a disulfide bond; Tyr at position 17 is amidated. |

```
Tyr Cys Ser Lys Xaa Arg His Cys Ile Asn Leu Ile Thr Arg Gln
                 5                   10                  15

Arg Tyr
```

## Claims

1. A peptide derivative of the formula

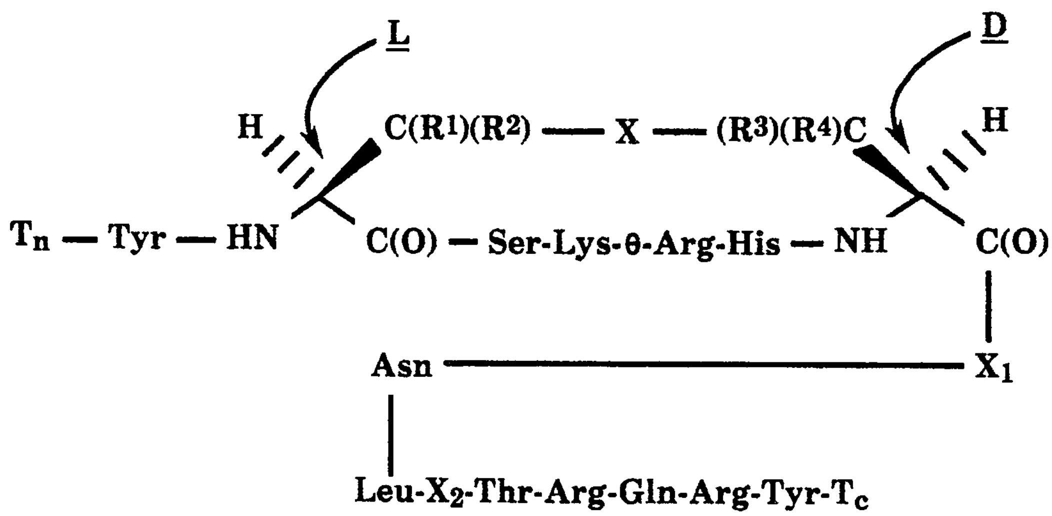

1

wherein $X_1$ is Leu, Ile, Met, Nle, or Val;
$X_2$ is Leu, Ile, Met, Nle, or Val;
$R^1$, $R^2$, $R^3$, and $R^4$ are each independently selected from a hydrogen and $(C_1\text{-}C_4)$alkyl;
$T_n$ is $(C_2\text{-}C_{10})$acyl, $(C_1\text{-}C_4)$alkyl;
$T_c$ is OR' or NHR';
wherein R' is a hydrogen or a $(C_1\text{-}C_4)$alkyl group;
$\theta$ is a group of the structural formula

$-NH-(CH_2)_n-CO_2-$;

wherein n is an integer of from 1-11; and
X is a bond or is a group of the formulae

-S-S-,

-S-,

-O-,

$-S-(C(R^5)(R^6))_m-S-$,

$-O-(C(R^5)(R^6))_m-O-$,

$-S-(C(R^5)(R^6))_m-O-$,

$-O-(C(R^5)(R^6))_m-S-$,

-C(=O)-N(R')-,

-N(R')-C(=O)-,

-C(=O)-O-,

-O-C(=O)-,

C(=O)-S-,

-S-C(=O)-, and

-N(H)-C(=O)-N(H)-

wherein $R^5$ and $R^6$ are each independently selected from the group consisting of hydrogen and ($C_1$-$C_4$)alkyl and m is an integer of a value of from 1 to 3
or a pharmaceutically acceptable salt thereof.

2. A peptide derivative of claim 1 wherein $X_1$ is isoleucine.

3. A peptide derivative of claim 1 wherein $X_2$ is isoleucine.

4. A peptide derivative of claim 1 wherein $X_1$ and $X_2$ are each isoleucine.

5. A peptide derivative of claim 1 wherein *θ* is Aoc.

6. A peptide derivative of claim 5 wherein $X_1$ and $X_2$ are each isoleucine.

7. A peptide derivative of claim 1 wherein $T_c$ is $-NH_2$.

8. A peptide derivative of claim 1 which is

Tyr-Cys-Ser-Lys-Aoc-Arg-His-cys-Ile-Asn-Leu-Ile-Thr-Arg-Gln-Arg-Tyr-$NH_2$

**9.** A peptide derivative of one of claims 1 - 8 for use in a method of treating diarrhea, especially secretory diarrhea and the diarrheas associated with carcinoid syndrome and VIPomas.

**10.** A pharmaceutical composition which comprises a peptide derivative of one of claims 1 - 8 and a physiologically acceptable carrier.

**Claims for the following Contracting State: ES**

**1.** A method for producing a peptide derivative of the formula

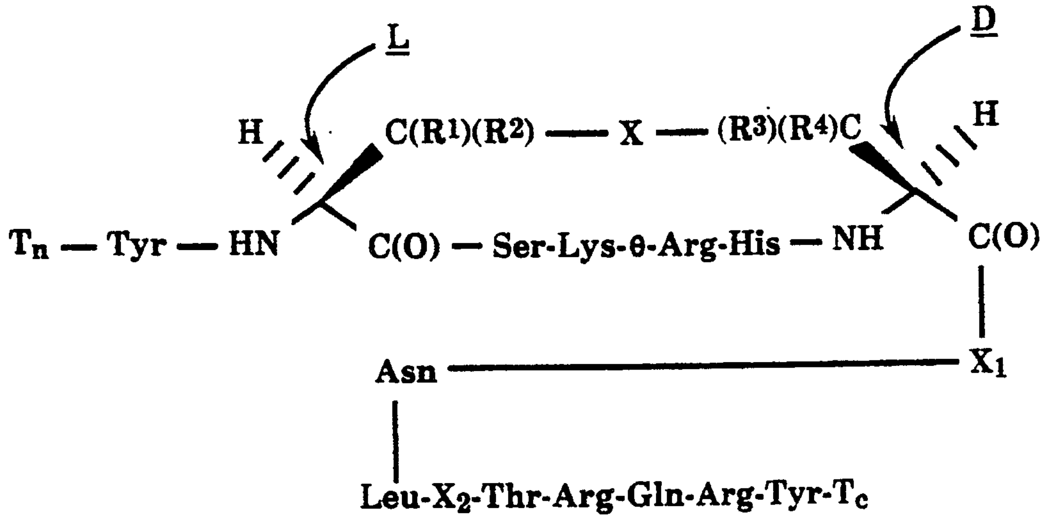

1

wherein $X_1$ is Leu, Ile, Met, Nle, or Val;
$X_2$ is Leu, Ile, Met, Nle, or Val;
$R^1$, $R^2$, $R^3$, and $R^4$ are each independently selected from a hydrogen and $(C_1\text{-}C_4)$alkyl;
$T_n$ is $(C_2\text{-}C_{10})$acyl, $(C_1\text{-}C_4)$alkyl;
$T_c$ is OR' or NHR';
wherein R' is a hydrogen or a $(C_1\text{-}C_4)$alkyl group;
$\theta$ is a group of the structural formula

$-NH-(CH_2)_n-CO_2-$;

wherein n is an integer of from 1-11; and
X is a bond or is a group of the formulae

-S-S-,

-S-,

-O-,

$-S-(C(R^5)(R^6))_m-S-$,

$-O-(C(R^5)(R^6))_m-O-$,

$-S-(C(R^5)(R^6))_m-O-$,

$-O-(C(R^5)(R^6))_m-S-$,

-C(=O)-N(R')-,

-N(R')-C(=O)-,

-C(=O)-O-,

-O-C(=O)-,

C(=O)-S-,

-S-C(=O)-, and

-N(H)-C(=O)-N(H)-

wherein $R^5$ and $R^6$ are each independently selected from the group consisting of hydrogen and $(C_1-C_4)$alkyl and m is an integer of a value of from 1 to 3
or a pharmaceutically acceptable salt thereof,
which comprises carrying out a per se known peptide synthesis e.g. by a solid phase sequential procedure using established automated methods such as by use of an automated peptide synthesizer.

2. The method of claim 1 wherein $X_1$ is isoleucine.

3. The method of claim 1 wherein $X_2$ is isoleucine.

4. The method of claim 1 wherein $X_1$ and $X_2$ are each isoleucine.

5. The method of claim 1 wherein $\theta$ is Aoc.

6. The method of claim 5 wherein $X_1$ and $X_2$ are each isoleucine.

7. The method of claim 1 wherein $T_c$ is $-NH_2$.

8. The method of claim 1 which is

**Tyr-Cys-Ser-Lys-Aoc-Arg-His-cys-Ile-Asn-Leu-Ile-Thr-Arg-Gln-Arg-Tyr-$NH_2$**

9. A method for preparing a pharmaceutical composition which comprises combining a peptide derivative obtained according to one of claims 1 - 8 with a physiologically acceptable carrier.

**Claims for the following Contracting State: GR**

1. A peptide derivative of the formula

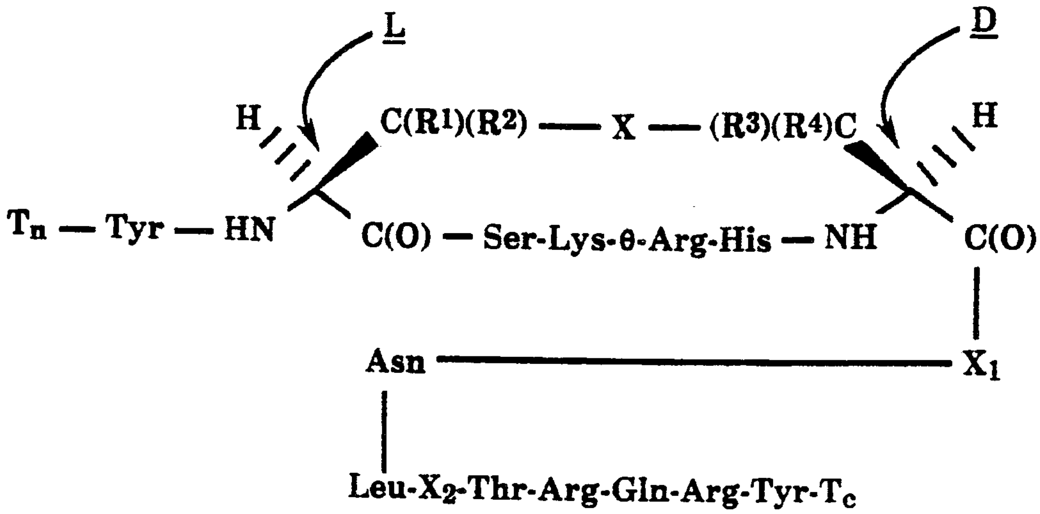

1

wherein $X_1$ is Leu, Ile, Met, Nle, or Val;
$X_2$ is Leu, Ile, Met, Nle, or Val;
$R^1$, $R^2$, $R^3$, and $R^4$ are each independently selected from a hydrogen and $(C_1-C_4)$alkyl;
$T_n$ is $(C_2-C_{10})$acyl, $(C_1-C_4)$alkyl;
$T_c$ is OR' or NHR';
wherein R' is a hydrogen or a $(C_1-C_4)$alkyl group;
$\theta$ is a group of the structural formula

$-NH-(CH_2)_n-CO_2-$;

wherein n is an integer of from 1-11; and
X is a bond or is a group of the formulae

$-S-S-,$

$-S-,$

$-O-,$

$-S-(C(R^5)(R^6))_m-S-,$

$-O-(C(R^5)(R^6))_m-O-,$

$-S-(C(R^5)(R^6))_m-O-,$

$-O-(C(R^5)(R^6))_m-S-,$

$-C(=O)-N(R')-,$

$-N(R')-C(=O)-,$

$-C(=O)-O-,$

$-O-C(=O)-,$

$C(=O)-S-,$

$-S-C(=O)-,$ and

$-N(H)-C(=O)-N(H)-$

wherein $R^5$ and $R^6$ are each independently selected from the group consisting of hydrogen and ($C_1$-$C_4$)alkyl and m is an integer of a value of from 1 to 3
or a pharmaceutically acceptable salt thereof.

2. A peptide derivative of claim 1 wherein $X_1$ is isoleucine.

3. A peptide derivative of claim 1 wherein $X_2$ is isoleucine.

4. A peptide derivative of claim 1 wherein $X_1$ and $X_2$ are each isoleucine.

5. A peptide derivative of claim 1 wherein $\theta$ is Aoc.

6. A peptide derivative of claim 5 wherein $X_1$ and $X_2$ are each isoleucine.

7. A peptide derivative of claim 1 wherein $T_c$ is $-NH_2$.

8. A peptide derivative of claim 1 which is

Tyr-Cys-Ser-Lys-Aoc-Arg-His-cys-Ile-Asn-Leu-Ile-Thr-Arg-Gln-Arg-Tyr-$NH_2$

9. A method for preparing a pharmaceutical composition which comprises combining a peptide derivative according to one of claims 1 - 8 with a physiologically acceptable carrier.